(19) **Europäisches Patentamt**
**European Patent Office**
**Office européen des brevets**

(11) **EP 2 903 672 B1**

(12) **EUROPÄISCHE PATENTSCHRIFT**

(45) Veröffentlichungstag und Bekanntmachung des Hinweises auf die Patenterteilung:
**10.01.2018 Patentblatt 2018/02**

(21) Anmeldenummer: **13782960.2**

(22) Anmeldetag: **02.10.2013**

(51) Int Cl.:
***A61M 15/00*** *(2006.01)*

(86) Internationale Anmeldenummer:
**PCT/EP2013/002958**

(87) Internationale Veröffentlichungsnummer:
**WO 2014/053242 (10.04.2014 Gazette 2014/15)**

(54) **SYSTEM, VERFAHREN UND VERWENDUNGZUM TRAINIEREN EINES INHALATIONSVORGANGS**

SYSTEM, METHOD AND USE FOR TRAINING AN INHALATION PROCESS

SYSTÈME, PROCÉDÉ ET UTILISATION AUX FINS D'ENTRAÎNEMENT À UNE OPÉRATION D'INHALATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.10.2012 EP 12006897**
**22.10.2012 EP 12007259**

(43) Veröffentlichungstag der Anmeldung:
**12.08.2015 Patentblatt 2015/33**

(73) Patentinhaber: **Boehringer Ingelheim International GmbH**
**55216 Ingelheim am Rhein (DE)**

(72) Erfinder:
- **ADAMS, Patricia**
**55216 Ingelheim am Rhein (DE)**
- **FRANK, Marion**
**55216 Ingelheim am Rhein (DE)**
- **WACHTEL, Herbert**
**55216 Ingelheim am Rhein (DE)**

(74) Vertreter: **Von Rohr Patentanwälte Partnerschaft mbB et al**
**Rüttenscheider Straße 62**
**45130 Essen (DE)**

(56) Entgegenhaltungen:
**WO-A1-2007/101438        WO-A1-2011/056889**
**WO-A1-2011/083377        WO-A2-2006/008745**
**WO-A2-2010/023591        WO-A2-2011/153406**
**DE-U1-202008 010 475    DE-U1-202011 106 292**
**US-A1- 2011 226 236      US-A1- 2012 116 241**

- **WOLFORD HOSH: "SpiroSmart App Turns Your iPhone into an Accurate Spirometer", INTERNET CITATION, 20. September 2012 (2012-09-20), Seiten 1-2, XP002699445, Gefunden im Internet: URL:http://www.webpronews.com/spirosmart-a pp-turns-your-iphone-into-an-accurate-spir ometer-2012-09 [gefunden am 2013-06-25]**

**Beschreibung**

Die vorliegende Erfindung betrifft ein Inhalationstrainingssystem zum Trainieren eines Medikamenteninhalationsvorgangs eines Patienten gemäß dem Oberbegriff des Anspruchs 1, ein Verfahren zum Trainieren eines Medikamenteninhalationsvorgangs eines Patienten ohne Abgabe einer Medikamentendosis gemäß dem Oberbegriff des Anspruchs 7 und eine Verwendung eines portablen Kommunikationsgeräts zum Trainieren eines Medikamenteninhalationsvorgangs eines Patienten gemäß Anspruch 9.

Zu inhalierende Medikamente stellen eine bevorzugte Therapie für Patienten mit Asthma, einer chronisch obstruktiven Lungenerkrankung oder anderen chronischen oder akuten Atemwegserkrankungen dar.

Zur Inhalation von Medikamenten werden sogenannte Inhalatoren verwendet. Die am häufigsten gebrauchten Inhalatoren sind druckbeaufschlagte Inhalatoren mit abgemessener Dosierung (pressurized metered-dose inhalers, pMDIs) und Trockenpulverinhalatoren (dry-powder inhalers, DPIs). pMDIs wurden entwickelt, um beim Einatmen des Patienten eine präzise Menge bzw. Dosis eines Medikaments in Form einer Wolke aus Aerosoltröpfchen der Lunge des Patienten zuzuführen. Trockenpulverinhalatoren sind derart ausgebildet, dass sie beim Einatmen des Patienten eine abgemessene Menge an trockenen, pulverisierten Teilchen der Lunge zuführen.

Die Effektivität zu inhalierender Medikamente hängt in hohem Maße von der Anwendungsweise des Inhalators durch den Patienten ab. Optimalerweise gelangt die richtige Menge des Medikaments zum richtigen Zeitpunkt zu den gewünschten Bereichen der Lunge. Anderenfalls wird der therapeutische Effekt vermindert und/oder das Risiko gegenteiliger Effekte erhöht.

In der Literatur finden sich zahlreiche Belege dafür, dass viele Patienten Inhalatoren falsch verwenden. Eine Unterweisung des Patienten hinsichtlich einer korrekten Inhalationstechnik kann die Nutzung von Inhalatoren verbessern. Neben schriftlichen und mündlichen Anweisungen sind hierzu praktische Übungen hilfreich.

Da das Atmen in der Regel unterbewusst abläuft und sich im Laufe eines Lebens entwickelt, ist es allerdings für einen Patienten besonders schwierig, seine Inhalation zu verändern, um die Effektivität eines zu inhalierenden Medikaments zu erhöhen. Vielmehr ist es bekannt, dass viele Patienten bereits kurze Zeit nach einer Unterweisung wieder eine suboptimale Inhalation anwenden. Deshalb wird ein wiederholtes, vorzugsweise regelmäßiges Trainieren der Inhalation und dessen Überprüfung empfohlen.

Zu diesem Zweck wurden Inhalationstrainingssysteme entwickelt. Mit diesen Systemen kann von Patienten die Inhalation von Medikamenten mittels eines Inhalators trainiert werden. Dabei können diese Systeme auf dem Markt erhältliche Inhalatoren oder Nachbildungen von Inhalatoren oder Teilen davon einsetzen. Bekannte Inhalationstrainingssysteme unterscheiden sich unter anderem hinsichtlich des Inhalatormodells, für das der Patient trainiert werden soll, hinsichtlich der Art der Rückkopplung an den Patienten (z. B. akustisch oder visuell), hinsichtlich der Messgröße (z. B. inhaliertes Volumen, beim Inhalieren erzeugter Volumenstrom bzw. Durchfluss oder Massenstrom, Geschwindigkeit der inhalierten Partikel während des Inhalationsvorgangs), hinsichtlich der Sensorik und Aktorik (z. B. mechanisch, magnetisch oder elektronisch) sowie hinsichtlich Größe, Handhabung und Kosten.

Die WO 2010/023591 A2, die den Ausgangspunkt der vorliegenden Erfindung bildet, zeigt ein Inhalationstrainingssystem mit einem Inhalator, einem Gehäuse für den Inhalator, einem Mundstück und einer Wandlereinrichtung. Die Wandlereinrichtung ist zur Umwandlung der Luftströmung, die bei einem Inhalationsvorgang eines Patienten in dem Mundstück auftritt, in ein akustisches Signal ausgebildet.

An dem Gehäuse des bekannten Inhalationstrainingssystems ist ein Tongenerator angebracht, der bei Auftreten eines oder mehrerer Ereignisse hörbare Instruktionen für den Patienten bereitstellen kann. Ein entsprechendes Ereignis kann durch einen Lärmerzeuger wie z. B. eine Pfeife hervorgerufen werden. Der Lärmerzeuger befindet sich in dem Gehäuse und wird durch die Luftströmung beim Inhalieren ausgelöst. Mit Hilfe eines Sensors wie z. B. einem Mikrofon wird der erzeugte Lärm erfasst.

Die US 2012/0116241 A1 zeigt ein portables Asthmadetektionsgerät mit einem Gehäuse, einem Gasdetektionsmodul, einem Gaseinleitrohr und einer Signalübermittlungseinheit. Zur Bestimmung des Zustandes der Atmung eines Patienten muss dieser in einen Eingang des Gaseinleitrohrs exhalieren. Das exhalierte Gas versetzt eine Turbine des Gasdetektionsmoduls in Rotation, welche mit einem Luftstromsensor detektiert wird. Das portable Asthmadetektionsgerät kann mit einem Smartphone kooperieren, das zur Datenverarbeitung, Drahtlosübertragung, Positionsbestimmung und Ergebnisanzeige in Echtzeit dient.

Die GB 2 479 953 A zeigt ein Inhalator mit einem Gehäuse, einem Mundstück und einem im Gehäuse montierten Mikrofon. Hierbei wird eine im Mundstück auftretende Luftströmung nicht in ein akustisches Signal umgewandelt, sondern die beim Inhalieren auftretenden Geräusche im Luftkanal werden direkt mittels des Mikrofons aufgenommen. Anschließend wird die Energie dieses Aufnahmesignals analysiert und der Patient zu Trainingszwecken mit entsprechenden hörbaren oder sichtbaren Informationen zum Inhalationsvorgang versorgt.

Die EP 0 933 092 A1 zeigt ein Inhalationstrainingsgerät mit einem Inhalator und einem Flussratenmessgerät. Das Flussratenmessgerät hat einen Deflektor, der beim Inhalieren gebogen wird, und einen magnetischen Sensor, der die Biegung des Deflektors messen kann.

Die WO 97/13553 A1 zeigt ein Inhalationstrainingsgerät, das auf einen beliebigen MDI aufsetzbar ist. Die in einem Mundstück auftretende Luftströmung wird mit Hilfe eines Thermistors und eines Sensors gemessen.

Die US 5,839,429 A zeigt Inhalationstrainingsgerät mit einem Inhalator und einem Verarbeitungsgerät. Zur Messung des Inhalationsflusses misst ein im Inhalator positioniertes Mikrofon die von der Luftströmung beim Inhalieren erzeugten Geräusche.

Die WO 00/69496 A1 zeigt ein System zum Trainieren von Patienten hinsichtlich der korrekten Nutzung eines Inhalators. Zur Messung der beim Inhalieren auftretenden Luftströmung weist das System ein Druckänderungskanal zur Kanalisierung der Luftströmung und Erzeugung einer Druckänderung beim Inhalieren auf. Eine etwaige Druckänderung wird mittels eines Druckmessgeräts erfasst.

Die US 2010/192948 A1 zeigt ein Gerät zum lösbaren Anbringen an einen Inhalator, das die Nutzung des Inhalators durch einen Patienten überwacht. Dieses Gerät umfasst ein Gehäuse, einen optischen Zähler der Medikamentendosis und ein elektronisches Steuermodul. Das Steuermodul umfasst einen drahtlosen Sender und Empfänger, z. B. in Form eines Mobiltelefonchips, zur interaktiven Kommunikation mit externen elektronischen Geräten, wie z. B. einem Mobiltelefon.

Die US 2009/263773 A1 zeigt eine Vorrichtung für Atemübungen und Meditation mit einer grafischen Anzeige, Eingabeelementen und einer Steuerung. Die Vorrichtung kann als Software auf einem Personalcomputer implementiert werden.

Die WO 2011/056889 A1 zeigt ein Inhalationssimulationssystem zur Nutzung mit einem Inhalator. Das System umfasst ein Inhalationstrainingsgerät und ein Verarbeitungssystem. Das Verarbeitungssystem kann ein Kommunikationsgerät umfassen. Mit Hilfe eines Softwareprogramms auf dem Kommunikationsgerät kann eine grafische Benutzeroberfläche initialisiert und eine visuelle Startanzeige ausgewertet werden. Mittels eines Mikrofons werden direkt die Geräusche gemessen, die von einer in einem Mundstück auftretenden Luftströmung verursacht werden. Die Messwerte werden digitalisiert an einen Computer übertragen. Anhand der Messwerte lassen sich eine Inhalationszeit und eine inhalierte Medikamentendosis bestimmen.

Bekannt ist ferner ein Softwareprogramm "SpiroSmart" für ein Smartphone, um eine spirometrische Analyse durchzuführen. Dazu bläst ein Nutzer in ein Mikrofon des Smartphones.

Die US 2004/0187869 A1 zeigt ein dediziertes Inhalationstrainingssystem, das ein Mundstück und eine Wandlereinrichtung aufweist. Die Wandlereinrichtung wandelt eine Luftströmung, die bei einem Inhalationsvorgang eines Patienten in dem Mundstück auftritt, in einen Druckabfall bzw. Unterdruck um. Eine Steuerschaltung wandelt den Druckabfall bzw. Unterdruck in ein analoges, linearisiertes Signal um.

Die WO 2011/153406 A2 lehrt das Vorsehen eines Aktivierungselements (z. B. eines Mikrofons), das bei Detektion einer Exhalation eines Nutzers die Abgabe einer Medikamentendosis durch einen nasalen Vorsprung auslöst, damit die Abgabe der Medikamentendosis nur beim Ausatmen des Nutzer erfolgt.

Die WO 2006/008745 A2 zeigt ein Verfahren zur Analyse der Atmung eines Nutzers. Mittels eines berührungslosen Mikrofons wird ein Rohsignal erzeugt, das ein Luftströmungsgeräusch der Atmung des Nutzers indiziert. Das Rohsignal wird zur Bestimmung von Atmungsparametern analysiert.

Die US 2011/226236 A1 betrifft eine Inhalatorkomponente für die intermittierende, inhalations- oder zugsynchrone Bildung eines Dampf-Luft-Gemisches oder/und Kondensationsaerosols.

Die WO 2007/101438 A1 zeigt ein elektronisches Überwachungsgerät, das an einer äußeren Oberfläche eines konventionellen Inhalators befestigt ist. Der Inhalator dient zur Abgabe eines Medikaments in einer Abgabeposition. Zwischen der Abgabeposition und einem Ausgang ist ein Strömungspfad definiert. Wenn ein Nutzer das Medikament inhaliert, erzeugt der Strömungspfad im Inhalator ein charakteristisches Strömungsgeräusch. Ein Teil dieses Strömungsgeräuschs wird durch die massive Struktur des Inhalators übertragen. Das Überwachungsgerät umfasst Sensoren (z. B. ein Mikrofon), die die so übermittelten Geräusche oder Vibrationen an der äußeren Oberfläche des Inhalators im Frequenzbereich von 100 bis 3000 Hz erfassen können.

Aufgabe der vorliegenden Erfindung ist es, ein Inhalationstrainingssystem, ein Verfahren und eine Verwendung anzugeben, wobei ein effektives, einfaches, zuverlässiges und/oder kostengünstiges Trainieren eines Medikamenteninhalationsvorgangs eines Patienten mit elektronischer Rückkopplung an den Patienten und/oder einen Dritten und/oder ein einfacher, kostengünstiger und/oder robuster Aufbau und/oder die Verwendung von portablen Kommunikationsgeräten ermöglicht wird bzw. werden.

Die genannte Aufgabe wird durch ein Inhalationstrainingssystem gemäß Anspruch 1, ein Verfahren gemäß Anspruch 7 oder eine Verwendung gemäß Anspruch 9 gelöst. Bevorzugte Ausgestaltungen der Erfindung sind Gegenstand der jeweiligen Unteransprüche.

Gemäß einem Aspekt der vorliegenden Erfindung weist das Inhalationstrainingssystem ein elektronisches Gerät und eine Halteeinrichtung auf, die zur Halterung des elektronischen Geräts ausgebildet ist, so dass das elektronische Gerät das von einer insbesondere nur mechanisch arbeitenden Wandlereinrichtung bereitgestellte, insbesondere akustische Signal messen bzw. erfassen kann.

Das elektronische Gerät im Sinne der vorliegenden Erfindung ist ein von der Wandlereinrichtung vorzugsweise separates bzw. unabhängiges Gerät, das nicht mit der Wandlereinrichtung verbunden sein muss.

Vorzugsweise ist die Halteeinrichtung derart ausgebildet, dass das elektronische Gerät das Signal der Wandlereinrichtung mittels eines Mikrofons in ein Mikrofonsignal umwandeln kann. Ein Mikrofonsignal im Sinne der vorliegenden Erfindung ist das Ausgangssignals eines Mikrofons. Ein Mikrofon wandelt Schallwellen (z. B. eines akustischen Signals) bzw. die aus Schallwellen resultierende Luftbewegung in eine elektrische Signalspannung um. Ein Mikrofonsignal wird üblicherweise symmetrisch übertragen und mit einem Mikrofonvorverstärker spannungsverstärkt.

Die Haltervorrichtung dient zur Halterung des elektronischen Geräts in einer bestimmten, gewünschten Position, insbesondere einer wieder lösbaren Halterung, also ohne es dauerhaft zu befestigen. Mit Hilfe der erfindungsgemäßen Halteeinrichtung ist eine Ausrichtung des elektronischen Geräts zur Wandlereinrichtung durch den Patienten nicht erforderlich. Die erfindungsgemäße Halteeinrichtung ermöglicht vorzugsweise eine optimale Ausrichtung des elektronischen Geräts zur Wandlereinrichtung und vermeidet oder verringert Verschiebungen des elektronischen Geräts aus der gewünschten Position. Ein wiederholtes Neuausrichten durch den Patienten ist nicht erforderlich.

Dank der vorschlagsgemäßen Halteeinrichtung muss der Patient das elektronische Gerät außerdem nicht selbst in einer Hand oder beiden Händen halten.

Das vorschlagsgemäße Inhalationstrainingssystem ermöglicht ein effektives, komfortables und zuverlässiges Trainieren eines Inhalationsvorgangs. Ferner bietet die Halteeinrichtung einen Schutz des elektronischen Geräts, z. B. vor Beschädigungen.

Die erfindungsgemäße Wandlereinrichtung ist vorzugsweise nicht zur weiteren Verarbeitung, Auswertung und/oder Rückkopplung des Signals ausgebildet. Gemäß der vorliegenden Erfindung werden diese Schritte vorzugsweise nur durch das elektronische Gerät durchgeführt. Dazu misst oder erfasst das elektronische Gerät das Signal und wandelt es in ein Mikrofonsignal um.

Die erfindungsgemäße Ausbildung des Inhalationstrainingssystems ermöglicht einen einfachen, robusten und kostengünstigen Aufbau der Wandlereinrichtung, insbesondere als Pfeife, sowie eine elektronische Rückkopplung an den Patienten und/oder einen Dritten.

Das Inhalationstrainingssystem gemäß der vorliegenden Erfindung ermöglicht es, die Funktionalität des elektronischen Geräts, insbesondere für die Verarbeitung und/oder Auswertung des Signals und/oder für die Rückkopplung an den Patienten und/oder einen Dritten, auf einfache, zuverlässige und kostengünstige Weise nutzbar zu machen.

Gleichzeitig ermöglicht das vorschlagsgemäße Inhalationstrainingssystem, die Funktionalität des elektronischen Geräts auf einfache und kostengünstige Weise hinsichtlich des Trainierens eines Inhalationsvorgangs eines Patienten zu erweitern.

Gemäß einem weiteren Aspekt der vorliegenden Erfindung ist die Wandlereinrichtung, die zur Umwandlung einer in dem Mundstück auftretenden Luftströmung in ein akustisches Signal, nämlich ein hörbares Pfeifen ausgebildet ist und dazu eine Pfeife umfasst, dem Mundstück zugeordnet. Die Wandlereinrichtung ist nicht einem für das Inhalationstrainingssystem verwendeten Inhalator und auch nicht dem elektronische Gerät zugeordnet. Die Wandlereinrichtung befindet sich nicht in einem Inhalator und auch nicht in dem elektronischen Gerät. Vielmehr ist die Wandlereinrichtung in, an, vor und/oder hinter dem Mundstück angebracht.

Diese Ausgestaltung bietet den Vorteil, dass die Wandlereinrichtung einfach und schnell montiert und entfernt bzw. ausgewechselt werden kann. Ferner können übliche Inhalatoren zum Trainieren eingesetzt werden, ohne dass diese Inhalatoren baulich verändert bzw. in diese Inhalatoren zusätzliche Komponenten eingebaut werden müssen.

Das elektronische Gerät ist zur Umwandlung des akustischen Signals in ein Mikrofonsignal mittels eines Mikrofons ausgebildet.

Bei dem elektronischen Gerät handelt es sich um ein portables Kommunikationsgerät. Die Umwandlung des akustischen Signals in ein Mikrofonsignal erfolgt mittels eines Mikrofons des portablen Kommunikationsgeräts.

Ein weiterer Aspekt der vorliegenden Erfindung betrifft ein Verfahren zum Trainieren eines Inhalationsvorgangs eines Patienten. Gemäß diesem Aspekt wird eine bei einem Inhalationsvorgang des Patienten in einem Mundstück auftretende Luftströmung durch eine Pfeife einer Wandlereinrichtung in ein akustisches Signal, nämlich ein hörbares Pfeifen, umgewandelt und dieses akustische Signal elektronisch ausgewertet. Dazu wird das akustische Signal mittels eines Mikrofons eines elektronischen Geräts in ein Mikrofonsignal umgewandelt. Das Mikrofonsignal wird in dem elektronischen Gerät verarbeitet und/oder ausgewertet. Bei dem elektronischen Gerät handelt es sich um ein portables Kommunikationsgerät. Die Wandlereinrichtung ist in und/oder an dem Mundstück angebracht.

Das erfindungsgemäße Verfahren ermöglicht die Realisierung einer einfachen, robusten, zuverlässigen und kostengünstigen Wandlereinrichtung. Darüber hinaus ermöglicht das Verfahren ein effektives, einfaches, zuverlässiges und kostengünstiges Trainieren eines Inhalationsvorgangs eines Patienten.

Das Verfahren gemäß der vorliegenden Erfindung ermöglicht es ferner, die Funktionalität des elektronischen Geräts für das Trainieren eines Inhalationsvorgangs auf einfache, zuverlässige und kostengünstige Weise nutzbar zu machen. Gleichzeitig ermöglicht das vorschlagsgemäße Verfahren, die Funktionalität des elektronischen Geräts auf einfache und kostengünstige Weise hinsichtlich des Trainierens eines Inhalationsvorgangs eines Patienten zu erweitern.

Ein weiterer Aspekt der vorliegenden Erfindung betrifft eine Verwendung eines portablen Kommunikationsgeräts. Gemäß diesem Aspekt wird das portable Kommunikationsgerät zum Trainieren eines Inhalationsvorgangs eines Patienten verwendet.

Dabei wird eine bei einem Inhalationsvorgang des Patienten in einem Mundstück auftretende Luftströmung durch eine in und/oder an dem Mundstück angebrachte Wandlereinrichtung mit einer Pfeife in ein akustisches Signal, nämlich ein hörbares Pfeifen, und dieses mittels eines Mikrofons des portablen Kommunikationsgeräts in ein Mikrofonsignal umgewandelt. Das Mikrofonsignal wird dann durch das portable Kommunikationsgerät ausgewertet.

Portable Kommunikationsgeräte im Sinne der vorliegenden Erfindung sind insbesondere elektronische Geräte, die zur Erfassung, Eingabe, Übermittlung und/oder Ausgabe von Informationen ausgebildet und von einem Menschen leicht zu transportieren sind. Die Informationen können akustischer, visueller und/oder sonstiger sensorischer Natur sein. Typische Anwendungen portabler Kommunikationsgeräte sind Telefonie, Datenübertragung, Spielen, Text-, Tabellen-, Bildverarbeitung, Fotografie und Musikwiedergabe. Typische Beispiele portabler Kommunikationsgeräte sind Mobiltelefone, Smartphones, Tablet-PCs, Handhelds und PDAs.

Die erfindungsgemäße Verwendung des portablen Kommunikationsgeräts ermöglicht, die Funktionalität des portablen Kommunikationsgeräts auf einfache und kostengünstige Weise hinsichtlich des Trainierens eines Inhalationsvorgangs eines Patienten zu erweitern.

Gleichzeitig ermöglicht die vorschlagsgemäße Verwendung, die umfangreiche Funktionalität typischer portabler Kommunikationsgeräte, insbesondere hinsichtlich der Verarbeitung und Auswertung von Signalen sowie hinsichtlich der interaktiven, multimedialen Möglichkeiten zur Rückkopplung an den Patienten und/oder einen Dritten, für das Trainieren eines Inhalationsvorgangs eines Patienten nutzbar zu machen.

Aufgrund der weiten Verbreitung portabler Kommunikationsgeräte kann auch Patienten Zugang zu einem Inhalationstraining verschafft werden, die sich kein Spezialgerät nur zum Inhalationstraining anschaffen möchten. Da die Besitzer portabler Kommunikationsgeräte an deren Handhabung gewöhnt sind, ermöglicht die erfindungsgemäße Verwendung auch ein leichteres und schnelleres Erlernen eines optimalen Inhalationsvorgangs. Da viele Menschen ständig mindestens ein portables Kommunikationsgerät mit sich tragen, kann die erfindungsgemäße Verwendung auch zu einem häufigeren, eventuell regelmäßigen Inhalationstraining führen.

Ferner erhöht die vorschlagsgemäße Verwendung den Bedienkomfort und Portabilität eines Inhalationstrainings.

Ein weiterer Aspekt der vorliegenden Erfindung betrifft ein Informationsspeichermedium, insbesondere für ein portables Kommunikationsgerät. Auf dem erfindungsgemäßen Informationsspeichermedium sind Instruktionen gespeichert, die bei ihrer Ausführung durch einen Prozessor das Durchführen vorzugsweise folgender Schritte bewirken:

- Initialisieren einer grafischen Benutzeroberfläche,
- Auswertung einer visuellen Startanzeige,
- Auslesen eines elektrischen Signalwertes eines akustischen Signalaufnehmers,
- Digitalisierung und Speicherung des elektrischen Signalwertes und
- Bestimmen einer effektiven Inhalationszeit und/oder inhalierten Medikamentendosis.

Das erfindungsgemäße Informationsspeichermedium ermöglicht ein effektives, einfaches, zuverlässiges und kostengünstiges Trainieren eines Inhalationsvorgangs eines Patienten. Darüber hinaus gestattet das erfindungsgemäße Informationsspeichermedium, das erfindungsgemäße Verfahren und/oder die vorschlagsgemäße Verwendung auf effektive, einfache und/oder kostengünstige Weise zu realisieren.

Vor einer Beschreibung der Zeichnung werden nachfolgend einige Begriffe näher erläutert.

Der Begriff "Inhalationsvorgang" umfasst erfindungsgemäß vorzugsweise ein Einatmen des Patienten, wobei das Einatmen über einen kurzen Zeitraum unterbrochen sein kann, also mehrere, kurz aufeinanderfolgende Einatemzüge umfassen kann. Weiter kann ein Inhalationsvorgang auch ein Anhalten der Luft bzw. des Einatmens und/oder ein Ausatmen und/oder ein Husten des Patienten umfassen.

Der Begriff "Patient" bezeichnet erfindungsgemäß vorzugsweise einen Menschen, der einen Inhalator einsetzen muss und/oder möchte, insbesondere einen Menschen, der an einer Erkrankung der Atemwege, ganz besonders an Asthma oder einer chronisch obstruktiven Lungenerkrankung, leidet und die Erkrankung mittels eines Inhalators therapiert.

Unter dem Begriff "Luftströmung" ist im Sinne der vorliegenden Erfindung vorzugsweise eine messbare, strömende Bewegung der Luft mit oder ohne Turbulenzen zu verstehen. Der Begriff "Luftströmungsverbindung" bezeichnet erfindungsgemäß vorzugsweise eine solche Verbindung für strömende Luft, durch die zumindest ein Teil einer Luftströmung strömen kann.

Die genannten Aspekte und Merkmale der vorliegenden Erfindung sowie die sich aus der weiteren Beschreibung und den Ansprüchen ergebenden Aspekte und Merkmale der Erfindung können unabhängig voneinander, aber auch in beliebiger Kombination realisiert werden.

Weitere Vorteile, Merkmale, Eigenschaften und Aspekte der vorliegenden Erfindung ergeben sich aus den Ansprüchen und der folgenden Beschreibung bevorzugter Ausführungsformen anhand der Zeichnung. In der Zeichnung zeigt:

Fig. 1 schematisch eine perspektivische Ansicht einer ersten bevorzugten Ausführungsform eines erfindungsgemäßen Inhalationstrainingssystems mit einem elektronischen Gerät;

Fig. 2     schematisch eine Seitenansicht des Inhalationstrainingssystems gemäß Fig. 1, jedoch ohne Mundstück und ohne elektronisches Gerät;

Fig. 3     schematisch einen Teilschnitt entlang Linie III-III von Fig. 2;

Fig. 4     schematisch eine Frontansicht einer zweiten bevorzugten Ausführungsform des erfindungsgemäßen Inhalationstrainingssystems;

Fig. 5     schematisch eine perspektivische Ansicht einer dritten bevorzugten Ausführungsform des erfindungsgemäßen Inhalationstrainingssystems mit einem elektronischen Gerät;

Fig. 6     schematisch eine Frontansicht des Inhalationstrainingssystems gemäß Fig. 5, jedoch ohne elektronisches Gerät;

Fig. 7     schematisch eine Rückansicht des Inhalationstrainingssystems gemäß Fig. 6; und

Fig. 8     schematisch ein Flussdiagramm eines bevorzugten Ablaufs bzw. einer App, der bzw. die insbesondere mit Hilfe eines erfindungsgemäßen Informationsspeichermediums ausgeführt werden kann.

In den Figuren werden für gleiche oder ähnliche Teile dieselben Bezugszeichen verwendet, wobei entsprechende Eigenschaften und Vorteile erreicht werden, auch wenn eine wiederholte Beschreibung weggelassen ist.

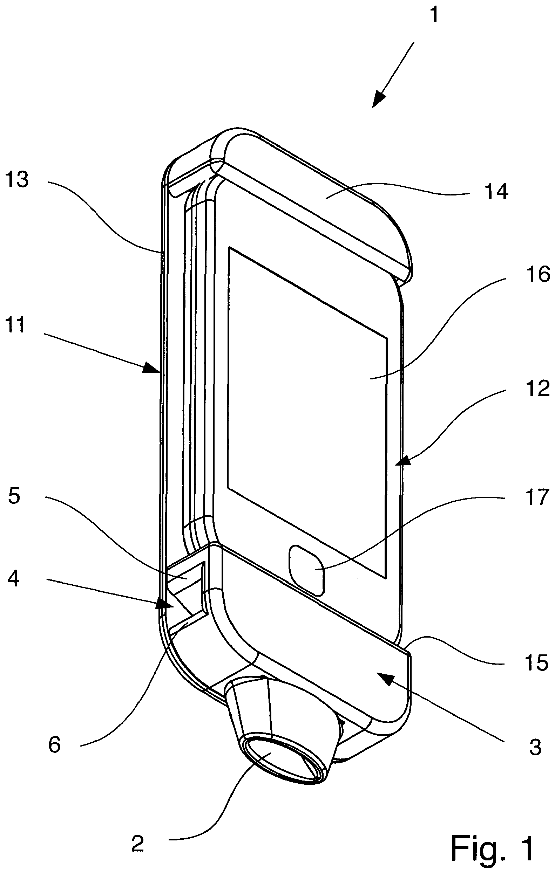

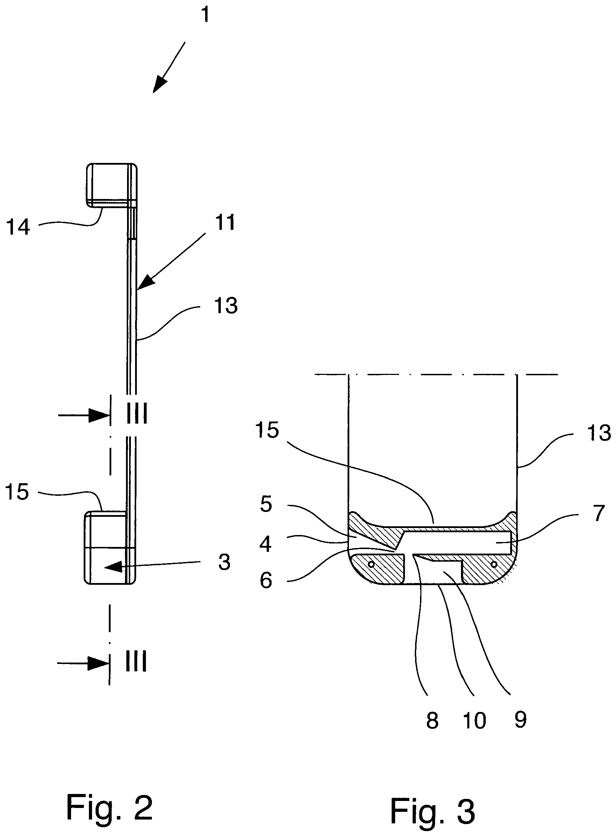

Fig. 1 zeigt eine erste bevorzugte Ausführungsform eines erfindungsgemäßen Inhalationstrainingssystems 1 mit einem elektronischen Gerät 12. Fig. 2 zeigt schematisch eine Seitenansicht, jedoch ohne Mundstück 2 und ohne elektronisches Gerät 12. Fig. 3 zeigt schematisch einen Teilschnitt gem. Linie III-III von Fig. 2.

Das Inhalationstrainingssystem 1 dient zum Trainieren eines Inhalationsvorgangs eines nicht dargestellten Patienten und weist ein Mundstück 2 und eine Wandlereinrichtung 3 auf.

Als Mundstück 2 können vorzugsweise verschiedene Arten von Mundstücken, insbesondere von Trockenpulverinhalatoren und/oder von druckbeaufschlagten Inhalatoren mit abgemessener Dosierung, bedarfsweise mit oder ohne Auslöser, eingesetzt werden.

Vorzugsweise ist das Mundstück 2 lösbar mit dem Inhalationstrainingssystem 1 bzw. der Wandlereinrichtung 3 verbunden und/oder wechselbar ausgebildet. Insbesondere ist das Mundstück 2 auf die Wandlereinrichtung 3 ein- oder aufgesteckt. Das Mundstück 2 kann aber auch beispielsweise mittels eines Gewindes, Magneten, Clips oder ähnlichem mit der Wandlereinrichtung 3 verbunden oder verbindbar sein.

Das Mundstück 2 steht zumindest während des Inhalationsvorgangs mit der Wandlereinrichtung 3 in einer Luftströmungsverbindung, so dass beim Inhalieren, insbesondere Einatmen, Luft durch das Mundstück 2 und - zumindest teilweise - auch durch die Wandlereinrichtung 3 strömt bzw. angesaugt wird.

Zur Benutzung nimmt der Patient das Mundstück 2 in den Mund. Bei einem Inhalationsvorgang, insbesondere beim Einatmen, strömt Luft durch die Wandlereinrichtung 3 und das Mundstück 2, es tritt also eine Luftströmung auf.

Die Wandlereinrichtung 3 ist zur Umwandlung der Luftströmung in ein nicht optisches, insbesondere akustisches Signal ausgebildet. Dazu umfasst die Wandlereinrichtung 3 vorzugsweise eine Pfeife oder ist als Pfeife ausgebildet. Die Pfeife bzw. Wandlereinrichtung 3 ist vorzugsweise derart ausgestaltet, dass ein Ansaugen bzw. Einatmen von Luft das akustische Signal und ggf. auch ein Ausatmen das gleiche akustische Signal oder ein anderes akustisches Signal (beispielsweise einen anderen Pfeifton) erzeugt.

Nachfolgend wird zunächst ein bevorzugter Aufbau der Wandlereinrichtung 3 anhand von Fig. 3 näher erläutert.

Vorzugsweise weist die Wandlereinrichtung 3 insbesondere seitlich einen Lufteinlass 4 auf. Ausgehend von dem Lufteinlass 4 wird beim Darstellungsbeispiel - insbesondere zwischen zwei entsprechend geformten Wandungen - ein sich vorzugsweise verjüngender Einlasskanal 5 gebildet, an den sich insbesondere über eine Drosselstelle 6 ein Resonanzraum 7 anschließt. Über den Lufteinlass 4 einströmende und durch den Einlasskanal 5 geführte Luft kann über eine vorzugsweise seitliche, insbesondere nach der Drosselstelle 6 bzw. im Resonanzrum 7 angeordnete Öffnung in einen Auslasskanal 9 strömen. Hierbei triff die Luftströmung vorzugweise schräg auf eine Kante 8 der Öffnung, so dass durch das Auftreffen der Luft auf die Kante 8 Turbulenzen an der Kante 8 entstehen, wodurch stehende Wellen in dem Resonanzraum 7 erzeugt werden. Die stehenden Wellen erzeugen das akustische Signal bzw. einen oder mehrere Töne, das bzw. die insbesondere über den Auslasskanal 9 oder einen Tonauslass 10 aus der Wandlereinrichtung 3 austritt bzw. austreten.

Vorzugsweise handelt es bei dem akustischen Signal um ein hörbares Signal und zwar insbesondere ein hörbares Pfeifen.

Aus dem Auslasskanal 9 bzw. Tonauslass 10 strömt die Luft dann in das sich anschließende - also in Luftströmungsverbindung stehende - Mundstück 2. Dies gilt beim Einatmen. Beim Ausatmen kann die Luft umgekehrt strömen und/oder ggf. einen anderen Strömungsweg nehmen. Weiter kann beim Ausatmen bedarfsweise auch kein akustisches Signal erzeugt werden.

Die Pfeife bzw. Wandlereinrichtung 3 ist vorzugsweise rein mechanisch und/oder nur aus feststehenden Teilen aufgebaut. Vorzugsweise ist die Pfeife bzw. Wandlereinrichtung 3 - zumindest weitestgehend - einstückig ausgebildet. So wird ein einfacher, robuster und kostengünstiger Aufbau der Wandlereinrichtung 3 ermöglicht.

Bedarfsweise kann die Wandlereinrichtung 3 auch mehrere Pfeifen aufweisen oder bilden. Diese können beispielsweise in Abhängigkeit von einer Luftströmungsrichtung und von einem Volumenstrom oder einer Strömungsgeschwindigkeit ansprechen und Töne bzw. Signale erzeugen. Die Töne bzw. Signale der verschiedenen Pfeifen sind dann vorzugsweise unterschiedlich, beispielsweise hinsichtlich Frequenz, Spektrum, Hörbarkeit und/oder Lautstärke. Auf diese Weise lassen sich vorzugsweise das Ein- und Ausatmen des Patienten signalisieren und daher entsprechend erfassen und auswerten.

Alternativ oder zusätzlich kann das Mundstück 2 auch mit der Wandlereinrichtung 3 derart zusammen wirken, dass in Abhängigkeit von dem jeweiligen Mundstück 2 bzw. dessen Eigenschaften (bspw. Strömungswiderstand) ein charakteristisches akustisches Signal (z. B. ein bestimmter Pfeifton) von der Wandlereinrichtung 3 erzeugt wird, um so das Mundstück 2 bzw. charakteristische Eigenschaften des Mundstücks 2 zu signalisieren und deren Erfassung und Auswertung zu ermöglichen.

Die Pfeifen können auch auf bestimmte Arten von Mundstücken 2 angepasst sein. Insbesondere können die Pfeifen so ausgestaltet sein, dass unterschiedliche Arten von Mundstücken 2 eine Eigenschaft des erzeugten Pfeifens wie z. B. die Tonhöhe beeinflussen. Auf diese Weise kann eine Erkennung des jeweils eingesetzten Mundstücks 2 vorgenommen werden. Diese Information könnte dann bei einer Auswertung des Signals bzw. Pfeifens berücksichtigt werden, insbesondere um das Inhalationstraining effektiver durchzuführen.

Die genannte Signalisierung des jeweiligen Mundstücks 2 bzw. dessen Eigenschaften kann beispielsweise dadurch ermöglicht werden, dass die Wandlereinrichtung 3 bzw. deren Pfeife oder Signalerzeugung veränderbar oder einstellbar oder auswechselbar ist oder dass das Mundstück 2 den Resonanzraum 7 und/oder einen sonstigen Teil der Pfeife zumindest teilweise bildet. Alternativ oder zusätzlich kann das Mundstück 2 auch eine einzelne oder mehrere Pfeifen der Wandlereinrichtung 3 spezifisch oder unterschiedlich (teilweise oder vollständig) abdecken oder freigeben. Alternativ oder zusätzlich kann das Mundstück 2 auch die Wandlereinrichtung 3 bilden und/oder zusammen mit dieser wechselbar sein.

Vorzugsweise weist das Inhalationstrainingssystem 1 eine Halteeinrichtung 11 auf, die zur Halterung des elektronischen Geräts 12 ausgebildet ist, so dass das elektronische Gerät 12 das von der Wandlereinrichtung 3 erzeugte bzw. bereitgestellte Signal messen bzw. erfassen, in ein Mikrofonsignal umwandeln und insbesondere auswerten kann.

Vorzugsweise weist die Halteeinrichtung 11 eine Rückwand 13, einen ersten oder oberen Haltebereich 14 und einen zweiten oder unteren Haltebereich 15 auf.

Die Haltebereiche 14, 15 weisen beim Darstellungsbereich jeweils vorzugsweise eine Aussparung, Vertiefung o. dgl. auf, um das elektronische Gerät 12 formschlüssig und/oder insbesondere gegenüberliegende Seiten oder Endbereiche des elektronischen Geräts 12 aufnehmen, halten oder umgreifen zu können.

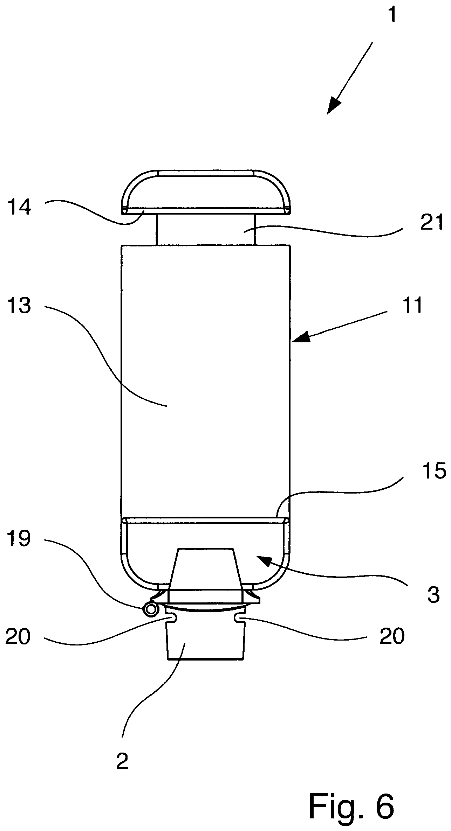

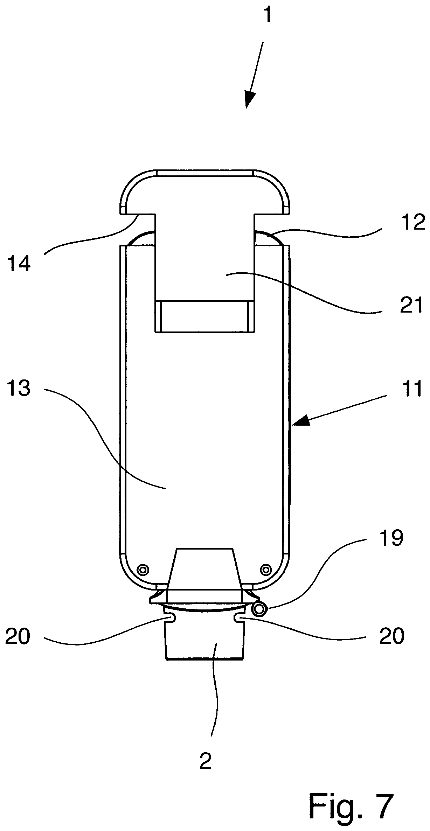

Die Haltebereiche 14, 15 sind vorzugsweise relativ zueinander beweglich, verstellbar oder verschiebbar, um das elektronische Gerät 12 aufnehmen und ggf. auch wieder lösen zu können. Die Halteeinrichtung 11 bzw. Rückwand 13 weist hierzu vorzugsweise einen Teleskopauszug 21 (wie in der dritten Ausführungsform in Fig. 6 und 7 angedeutet) auf, so dass bspw. der obere Haltebereich 14 zum unteren Haltebereich 15 verschiebbar ist. Jedoch sind hierzu auch andere konstruktive Lösungen möglich. Beispielsweise kann die Halteeinrichtung 11 bzw. Rückwand 13 und/oder der Haltebereich 14, 15 hierzu auch flexibel oder elastisch ausgebildet sein.

Zur Aufnahme des elektronischen Geräts 12 wird vorzugsweise zunächst ein unterer Teil des elektronischen Geräts 12 in den unteren Haltebereich 15 eingesetzt und das elektronische Gerät 12 an die Rückwand 13 gelehnt bzw. gedrückt. Dann kann der obere Haltebereich 14 mittels des Teleskopauszugs 21 nach unten bzw. zum unteren Haltebereich 15 hin verschoben werden, bis er das elektronische Gerät 12 auch oben insbesondere formschlüssig hält bzw. umgreift.

Die Halteeinrichtung 11 bzw. der Teleskopauszug 21 ist vorzugsweise derart gestaltet, dass ein (ungewolltes) Lösen des elektronischen Geräts 12 verhindert wird. Hierzu kann bspw. eine Ratsche, ausreichende Schwergängigkeit oder Klemmung, ein Feststellmittel o. dgl. insbesondere bezüglich des Teleskopauszugs 21 vorgesehen sein. Die Halteeinrichtung 11 ist vorzugsweise derart ausgebildet, dass das elektronische Gerät 12 wieder lösbar ist.

In der eingesetzten Position umgreift also die Halteeinrichtung 11 das elektronische Gerät 12 an gegenüberliegenden Seiten. Dabei wird das elektronische Gerät 12 teilweise von der Halteeinrichtung 11 überdeckt. Allerdings werden ein Bildschirm 16, ein Bedienknopf oder Mikrofon 17 und gegebenenfalls Lautsprecher des elektronischen Geräts 12 nicht von der Halteeinrichtung 11 überdeckt, so dass für den Patienten insbesondere der Bildschirm 16 sichtbar sowie der Bedienknopf bzw. das Mikrofon 17 und gegebenenfalls Lautsprecher zugänglich sind. Dadurch ist z. B. ein Telefonieren mit dem elektronischen Gerät 12 in der eingesetzten Position ermöglicht. Ferner wird eine Umwandlung des akustischen Signals der Wandlereinrichtung 3 in ein Mikrofonsignal mittels des Mikrofons 17 nicht bzw. nicht wesentlich beeinträchtigt.

Die Halteeinrichtung 11 ist vorzugsweise an die Form und/oder Größe verschiedener elektronischer Geräte 12 anpassbar.

Vorzugsweise ist die Halteeinrichtung 11 zur lösbaren Halterung des elektronischen Geräts 12 ausgebildet. Das elektronische Gerät 12 kann insbesondere durch nach oben Schieben des oberen Haltebereichs 14 wieder entnommen werden.

Alternativ oder zusätzlich zu der bevorzugten formschlüssigen bzw. beschriebenen Halterung kann die Halteeinrichtung 11 auch mittels eines magnetischen oder elastischen Mechanismus zur lösbaren Halterung des elektronischen Geräts 12 ausgebildet sein.

Die Halteeinrichtung 11 ist vorzugsweise zur Positionierung bzw. Ausrichtung des elektronischen Geräts 12 relativ zur Wandlereinrichtung 3 ausgebildet sein, so dass das elektronische Gerät 12 bzw. dessen Mikrofon 17 das Signal insbesondere optimal oder zuverlässig messen kann.

Vorzugsweise bilden das Mundstück 2, die Wandlereinrichtung 3 und/oder die Halteeinrichtung 11 eine Baueinheit. Insbesondere sind die Halteeinrichtung 11 und die Wandlereinrichtung 3 nicht von einander lösbar.

Weiter vorzugsweise bestehen das Mundstück 2, die Wandlereinrichtung 3 und/oder die Halteeinrichtung 11 aus Kunststoff und/oder weisen keine elektronischen Komponenten auf.

Nachfolgend werden weitere Ausführungsformen anhand der weiteren Fig. erläutert, wobei aber insbesondere nur wesentliche Unterschiede erläutert werden, so dass die bisherigen Ausführungen und Erläuterungen insbesondere ergänzend oder entsprechend gelten.

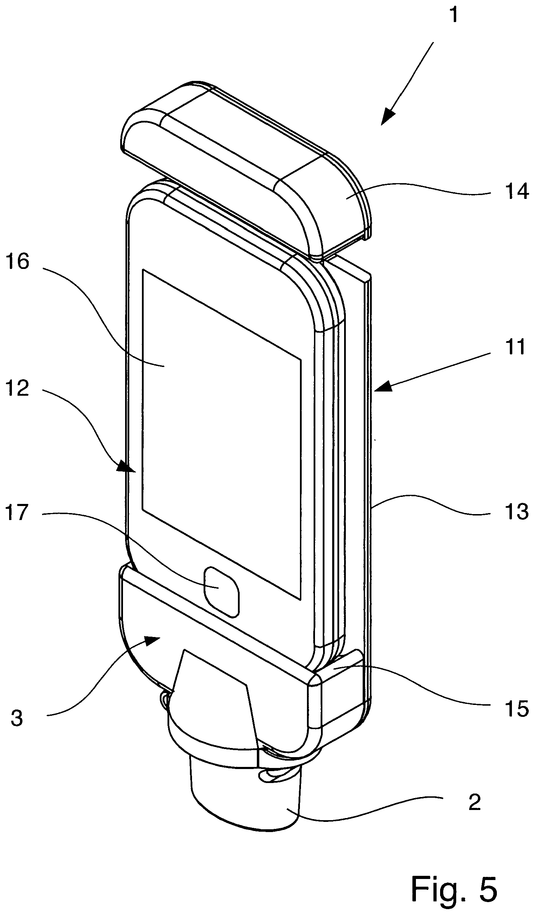

Fig. 4 zeigt schematisch eine Frontansicht einer zweiten bevorzugten Ausführungsform des erfindungsgemäßen Inhalationstrainingssystems 1. Insbesondere weist das Inhalationstrainingssystem 1 eine Schutzkappe 18 auf, die das Mundstück 2 im Nichtgebrauchszustand abdeckt und zur Benutzung insbesondere lösbar oder schwenkbar ist.

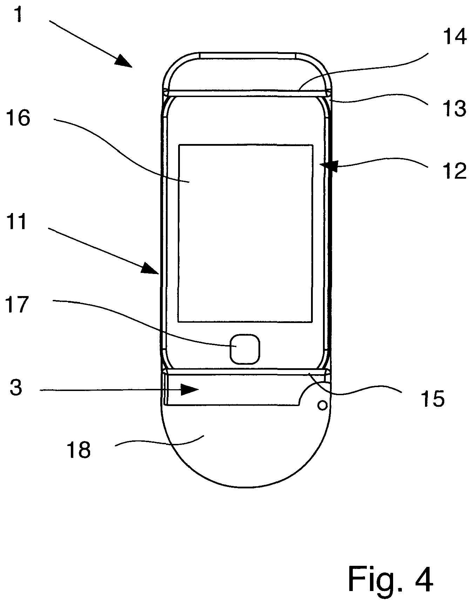

Fig. 5 bis 7 zeigen eine dritte bevorzugte Ausführungsform des erfindungsgemäßen Inhalationstrainingssystems 1. Fig. 5 zeigt das Inhalationstrainingssystem 1 mit dem elektronischen Gerät 12, wobei der obere Haltebereich 14 noch nicht auf das Gerät 12 aufgeschoben ist. Fig. 6 zeigt das Inhalationstrainingssystem 1 ohne das Gerät 12 in einer Frontansicht. Fig. 7 zeigt das Inhalationstrainingssystem 1 ohne das Gerät 12 in einer Rückansicht.

Bei der dritten Ausführungsform ist das Inhalationstrainingssystem 1 im Bereich des Mundstücks 2 etwas anders gestaltet. Insbesondere erstreckt sich das Mundstück 2 oder ein sich anschließender Halteabschnitt für den Patienten hier auf einer oder beiden Flachseiten seitlich über die Wandlereinrichtung 3 bzw. die Halteeinrichtung 11.

Fig. 6 und 7 deuten eine optionale Lagerung 19 für eine nicht dargestellte, insbesondere schwenkbare oder aufrastbare Schutzkappe 18 an. Die Lagerung 19 ist beispielsweise am Mundstück 2 gebildet.

Weiter kann das Mundstück 2 eine oder mehrere Zuluftöffnungen 20 aufweisen, durch die Luft beim Einatmen mit - also an der Wandlereinrichtung 3 vorbei - angesaugt werden kann.

Einzelne Aspekte und Merkmale der verschiedenen Ausführungsformen und die Ausführungsformen selbst können beliebig miteinander kombiniert, aber auch unabhängig voneinander realisiert werden.

Die nachfolgenden Ausführungen gelten für alle Ausführungsformen.

Bei dem elektronischen Gerät 12 handelt es sich insbesondere um ein portables Kommunikationsgerät, wie ein Telefon, Smartphone o. dgl.

Vorzugsweise ist das elektronische Gerät 12 zur Messung des von der Wandlereinrichtung 3 erzeugten bzw. bereitgestellten Signals, zur Messung von Werten der Signals, zur Verarbeitung gemessener Werte des Signals und/oder zur Auswertung bzw. Verarbeitung gemessener Werte des Signals ausgebildet.

Zur Messung bzw. Erfassung des Signals nimmt das elektronische Gerät 12 das Signal vorzugsweise mittels des Mikrofons 17 auf. Dann wandelt das elektronische Gerät 12 das erfasste Signal mittels des Mikrofons 17 in ein Mikrofonsignal um. Zur weiteren Verarbeitung des Mikrofonsignals ist das elektronische Gerät 12 vorzugsweise zur Digitalisierung des Mikrofonsignals ausgebildet. Vorzugsweise weist das elektronische Gerät 12 mindestens einen Mikroprozessor und/oder ein Programm zur Signalerfassung, -messung, -auswertung und/oder -anzeige auf.

Vorzugsweise ist das elektronische Gerät 12 zur Bestimmung oder Schätzung der Amplitude, Frequenz und/oder Phase des akustischen Signals ausgebildet.

Vorzugsweise ist das elektronische Gerät 12 zur Speicherung, Ausgabe und/oder insbesondere interaktiven Rückkopplung gemessener, verarbeiteter und/oder ausgewerteter Werte des Signals an den Patienten und/oder einen Dritten ausgebildet. Insbesondere weist das elektronische Gerät 12 eine Vorrichtung zur akustischen Rückkopplung der Auswertung, z. B. Lautsprecher, und/oder visuellen Rückkopplung der Auswertung, z. B. einen Bildschirm 16, auf.

Das elektronische Gerät 12 kann insbesondere Hinweise zur richtigen Inhalation und/oder Ratschläge zur Optimierung geben. Zur effektiveren Schulung des Patienten kann das elektronische Gerät 12 zusätzlich Bilder und/oder Videos anzeigen, die einen optimalen Inhalationsvorgang illustrieren.

Der Begriff "Interaktivität" bezeichnet dabei die Eigenschaften, dem Patienten zum individualisierten Lernen Eingriffs- und Steuerungsmöglichkeiten bereitzustellen. Dazu können beispielsweise die Auswahl und die Art der Darstellung von Informationen dem Vorwissen, den Interessen und Bedürfnissen des Patienten anpassbar bzw. von diesem manipulierbar sein. Ein ausschließliches Bereitstellen von Informationen stellt keine interaktive Rückkopplung im Sinne der vorliegenden Erfindung dar.

Vorzugsweise ist das elektronische Gerät 12 zur insbesondere drahtlosen Übertragung von gemessenen, verarbeiteten und/oder ausgewerteten Werten des Signals an ein anderes elektronisches Gerät ausgebildet. Auf diese Weise können solche Werte beispielsweise an einen Arzt übertragen werden, der auf dieser Basis eine Diagnose erstellen und/oder Ratschläge zur Verbesserung des Inhalationsvorgangs geben kann.

Vorzugsweise ist das elektronische Gerät 12 zur Ausführung zusätzlicher Anwendungen, insbesondere Telefonie, Datenübertragung, Spielen, Text-, Tabellen-, Bildverarbeitung, Fotografie oder Musikwiedergabe ausgebildet. Dadurch erhöht sich die Anwendbarkeit des erfindungsgemäßen Inhalationstrainingssystems 1.

Das Inhalationstrainingssystem 1 kann auch zur Überwachung eines Auslösens des Inhalationsvorgangs ausgebildet sein. So kann z. B. ausgewertet werden, ob der Inhalationsvorgang zum richtigen Zeitpunkt von dem Patienten ausgelöst wurde. Dadurch kann die Effektivität des Trainings erhöht werden.

Dazu kann beispielsweise das elektronische Gerät 12 so ausgebildet sein, dass eine Eingabe des Patienten, z. B. die Betätigung des Bedienknopfes 17, den Beginn des Inhalationsvorgangs markiert. Alternativ kann das Inhalationstrainingssystem 1, insbesondere das Mundstück 2, einen Auslöser aufweisen, wie er z. B. bei Inhalatoren eingesetzt wird. Der Auslöser kann dann so ausgebildet sein, dass ein Drücken und/oder Loslassen des Auslösers ein akustisches Signal erzeugt. Dieses akustische Signal kann von dem elektronischen Gerät 12 mittels eines Mikrofons 17 aufgenommen und anschließend von dem elektronischen Gerät 12 mit ausgewertet werden. Schließlich kann das elektronische Gerät 12 rückkoppeln, ob der Auslöser zum richtigen Zeitpunkt gedrückt und/oder losgelassen wurde und/oder ob das Inhalieren zeitlich richtig zum Auslösen erfolgte.

Das Inhalationstrainingssystem 1 ist vorzugsweise zum Trainieren einer möglichst optimalen effektiven Inhalationszeit $T_{in, eff}$ ausgebildet oder einsetzbar. Dadurch soll einem Patienten vorzugsweise das Erreichen einer möglichst optimalen effektiven Inhalationszeit ermöglicht werden. Bei einem Inhalator wird als effektive Inhalationszeit vorzugsweise die Zeit während eines Inhalationsvorgangs, insbesondere eines Einatmens, bezeichnet, in der der Inhalator eine Menge bzw. Dosis eines Medikaments abgibt. Insbesondere bezeichnet die effektive Inhalationszeit den Zeitanteil, in dem sich der Inhalationsvorgang und die Abgabe der Medikamentendosis überlappen.

Auf Basis der effektiven Inhalationszeit kann eine inhalierte Medikamentendosis (inhaled dose of drug, iDoD) bestimmt werden. Dies gilt insbesondere, wenn das Medikament mit einer konstanten Rate abgegeben wird. Die inhalierte Medikamentendosis kann als Prozentsatz der abgegebenen Medikamentendosis angegeben werden. Die effektive Inhalationszeit und die inhalierte Medikamentendosis haben eine besonders hohe Aussagekraft hinsichtlich der Güte des Inhalationsvorgangs.

Zum Trainieren der effektiven Inhalationszeit ist vorzugsweise das elektronische Gerät 12 zur Bestimmung der effektiven Inhalationszeit ausgebildet. Vorzugsweise weist hierbei das Mundstück 2 einen (nicht dargestellten) Auslöser auf. Alternativ kann der Auslöser beispielsweise durch den Bedienknopf 17 oder ein Drücken auf den Bildschirm 16 bzw. ein Betätigen einer visuellen Auslöseranzeige gebildet oder simuliert werden. Eine tatsächliche Abgabe einer Medikamentendosis muss dabei nicht erfolgen.

Zur Bestimmung der effektiven Inhalationszeit kann das elektronische Gerät 12 eine Abgabezeit bzw. Sprühzeit bestimmen. Mit Abgabezeit bzw. Sprühzeit ist dabei die Zeit gemeint, in der eine Abgabe einer Medikamentendosis durchgeführt oder simuliert wird. Insbesondere kann die Abgabezeit bzw. Sprühzeit als die Zeit bestimmt werden, die zwischen einem Betätigen des Auslösers zum Starten einer (tatsächlichen oder simulierten) Abgabe einer Medikamentendosis und dem Ende der Abgabe vergeht. Das Ende der Abgabe wird vorzugsweise durch eine fixe Abgabedauer bzw. Sprühdauer (spray duration, SDur) oder durch ein zweites, zeitlich nachgelagertes Betätigen des Auslösers festgelegt. Vorzugsweise wird die effektive Inhalationszeit in Prozent der Sprühdauer angegeben.

Bei einer bevorzugten Ausführungsform beträgt die effektive Inhalationszeit 0%, wenn die Abgabezeit außerhalb der Zeit des Inhalationsvorgangs bzw. des Inhalierens ($T_{in}$) liegt, d. h. wenn das einzige oder erste Betätigen des Auslösers nach Ende des Inhalationsvorgangs erfolgt oder wenn die Abgabezeit vor Beginn des Inhalationsvorgangs abgelaufen ist. Bei dieser Ausführungsform beträgt die effektive Inhalationszeit 100%, wenn die Abgabezeit vollständig innerhalb der Zeit des Inhalationsvorgangs liegt, d. h. wenn das einzige oder erste Betätigen des Auslösers nach Beginn des Inhalationsvorgangs erfolgt und die Abgabezeit vor Ende des Inhalationsvorgangs abgelaufen ist.

Beginnt die Abgabe vor Beginn des Inhalationsvorgangs und endet die Abgabe nach Ende des Inhalationsvorgangs, so wird die effektive Inhalationszeit vorzugsweise nach folgender Formel bestimmt:

$$T_{in, eff}[\%] = \frac{T_{in} \cdot 100}{SDur}.$$

Beginnt die Abgabe nach Beginn und vor Ende des Inhalationsvorgangs und endet die Abgabe nach Ende des Inhalationsvorgangs, so wird die effektive Inhalationszeit vorzugsweise nach folgender Formel bestimmt:

$$T_{in, eff}[\%] = \left(1 - \frac{\Delta + SDur - T_{in}}{SDur}\right) \cdot 100,$$

wobei Δ die Differenz zwischen Beginn der Abgabe und Beginn des Inhalationsvorgangs bezeichnet, d. h. Δ weist einen positiven Wert auf, wenn die Abgabe nach Beginn des Inhalationsvorgangs beginnt und Δ weist einen negativen Wert auf, wenn die Abgabe vor Beginn des Inhalationsvorgangs beginnt.

Beginnt die Abgabe vor Beginn des Inhalationsvorgangs und endet die Abgabe nach Beginn und vor Ende des Inhalationsvorgangs, so wird die effektive Inhalationszeit vorzugsweise nach folgender Formel bestimmt:

$$T_{in,eff}[\%] = \left( \frac{\Delta + SDur}{SDur} \right) \cdot 100,$$

wobei Δ wiederum die Differenz zwischen Beginn der Abgabe und Beginn des Inhalationsvorgangs bezeichnet.

Insbesondere können bei der Bestimmung der effektiven Inhalationszeit als Inhalationszeit $T_{in}$ ausschließlich Zeiten des Einatmens berücksichtigt werden. Wird das Einatmen also durch Luftanhalten oder Ausatmen unterbrochen, werden, vorzugsweise, diese Zeiten von der Inhalationszeit abgezogen.

Alternativ oder zusätzlich kann das elektronische Gerät 12 zur Bestimmung oder Schätzung eines beim Inhalationsvorgang erzeugten Volumenstroms bzw. Durchflusses und/oder einer dabei erzeugten Strömungsgeschwindigkeit ausgebildet sein. Diese beiden physikalischen Größen haben eine hohe Aussagekraft hinsichtlich der Güte des Inhalationsvorgangs.

Vorzugsweise weisen das Mundstück 2, die Wandlervorrichtung 3, die Halteeinrichtung 11 und/oder die Rückwand 13 mindestens eine Fläche zur Anbringung von Werbung und/oder Benutzungshinweisen auf.

Insgesamt ermöglicht das Inhalationstrainingssystem 1 auf einfache Weise eine nachhaltige Verbesserung des Inhalationsvorgangs, der Lungenfunktion und/oder der Lebensqualität des Patienten.

Bei dem vorschlagsgemäßen Verfahren wird eine bei dem Inhalationsvorgang des Patienten auftretende Luftströmung durch die Wandlereinrichtung 3 vorzugsweise in ein hörbares Pfeifen als akustisches Signal umgewandelt und dieses hörbare Pfeifen elektronisch ausgewertet.

Dazu wird das akustische Signal mittels eines Mikrofons eines portablen Kommunikationsgeräts 12 oder der Wandlereinrichtung 3 in ein Mikrofonsignal umgewandelt. Das Mikrofonsignal wird dann in dem portablen Kommunikationsgerät 12 verarbeitet und/oder ausgewertet.

Wird das akustische Signal mittels des Mikrofons der Wandlereinrichtung 3 in ein Mikrofonsignal umgewandelt, wird das Mikrofonsignal vorzugsweise an das portable Kommunikationsgerät 12 zur Verarbeitung und/oder Auswertung übertragen. Die Übertragung des Mikrofonsignals kann dabei kabelgebunden oder kabellos erfolgen.

Vorzugsweise wird das Pfeifen von einem elektronischen Gerät 12, insbesondere Kommunikationsgerät oder Telefon, erfasst bzw. gemessen. Vorzugsweise nimmt das elektronische Gerät 12 das Pfeifen mit Hilfe eines Mikrofons oder eines sonstigen geeigneten Sensors auf. Beispielsweise könnte die Wandlereinrichtung 3 alternativ oder zusätzlich ein optisches Signal bereitstellen, das von einer Kamera des elektronischen Geräts 12 erfasst wird.

Anhand aufgenommener Signale werden von dem elektronischen Gerät 12 vorzugsweise die effektive Inhalationszeit $T_{in, eff}$ und/oder die inhalierte Medikamentendosis iDoD wie oben beschrieben bestimmt. Alternativ oder zusätzlich kann das elektronische Gerät 12 einen beim Inhalationsvorgang erzeugten Volumenstrom und/oder eine dabei erzeugte Strömungsgeschwindigkeit schätzen. Das elektronische Gerät 12 nimmt also auch die elektronische Auswertung des Signals vor.

Vorzugsweise werden als Rückkopplung an den Patienten und/oder einen Dritten akustische und/oder visuelle Hinweise zur korrekten Inhalation und/oder zur Optimierung des Inhalationsvorgangs in insbesondere interaktiver Weise gegeben. Diese Ausgestaltung erhöht die Effektivität des Trainierens.

Vorzugsweise wird das Verfahren bzw. Trainieren der Inhalation mit einer Software durchgeführt, die an das Gerät 12 bzw. Kommunikationsgerät angepasst ist und insbesondere über ein Online-Portal bezogen und installiert werden kann. Typischerweise wird eine solche Software als "App" bezeichnet. Der Einsatz einer App verbessert die Flexibilität und den Bedienkomfort. Unter dem Begriff "App" ist vorzugsweise auch ein bevorzugter Verkehrsablauf bzw. ein bevorzugtes Verfahren zu verstehen.

Die App kann beispielsweise zur Verarbeitung und Interpretation des gemessenen Signals sowie zur Rückkopplung an den Patienten und/oder einen Dritten genutzt werden. Dazu kann die App mit Hilfe eines vorschlagsgemäßen Informationsspeichermediums ausgeführt oder bereitgestellt werden. Das Informationsspeichermedium ist vorzugsweise für den Einsatz in einem portablen Kommunikationsgerät ausgebildet, insbesondere optimiert im Hinblick auf Platzbedarf, Energieverbrauch, Zuverlässigkeit und Datenübertragungsrate.

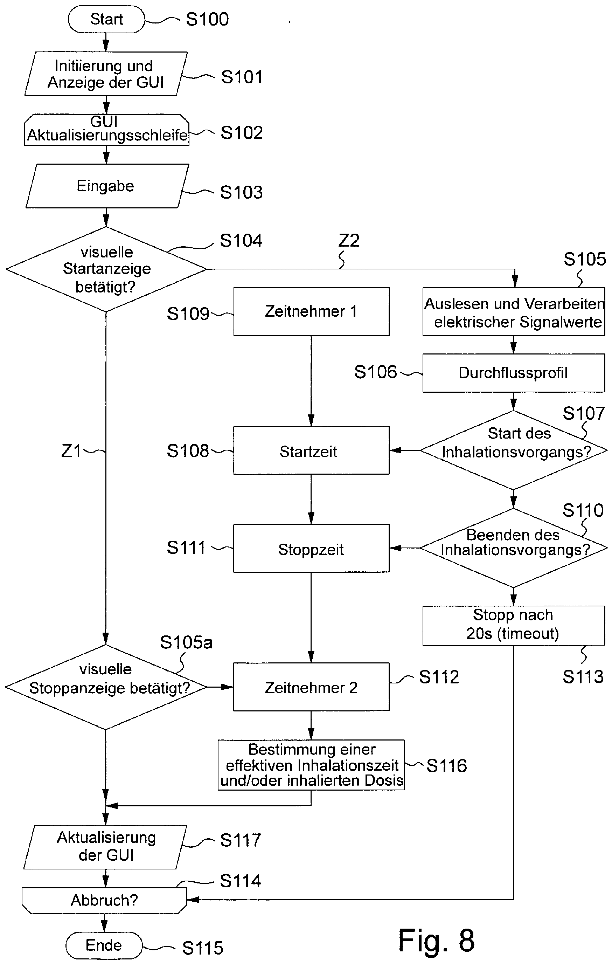

Auf dem Informationsspeichermedium sind vorzugsweise Instruktionen gespeichert, die bei ihrer Ausführung durch einen Prozessor das Durchführen nachfolgend beschriebener und in Fig. 8 in einem Flussdiagramm schematisch dargestellter Schritte bewirken. Fig. 8 stellt auch einen bevorzugten Verfahrensablauf dar.

Zu dem in Fig. 8 dargestellten und bevorzugten Verfahrensablauf bzw. zu den dargestellten Schritten der App können weitere Schritte hinzugefügt werden Es können auch einzelne Schritte des bevorzugten Ablaufs bzw. der App weggelassen werden. Die Reihenfolge der einzelnen Schritte kann verändert und verschiedene Schritte miteinander kombiniert werden.

Bei dem in Fig. 8 dargestellten und bevorzugten Verfahrensablauf bzw. den Schritten der App wird in einem ersten Schritt S100 die App bzw. das portable Kommunikationsgerät 12 gestartet.

In einem späteren Schritt S101 wird eine grafische Benutzeroberfläche (graphical user interface, GUI) initiiert und vorzugsweise auf dem Bildschirm 16 des portablen Kommunikationsgeräts 12 angezeigt. Dabei wird insbesondere auch eine visuelle Startanzeige bzw. eine visuelle Auslöseranzeige angezeigt.

In einem weiteren Schritt S102 wird eine Schleifenfunktion gestartet, mit deren Hilfe die GUI aktualisiert wird, um z. B. geänderte Inhalte der GUI anzuzeigen.

Vorzugsweise wird die Startanzeige bzw. Auslöseranzeige mit Hilfe der App bzw. des portablen Kommunikationsgeräts 12 ausgewertet. Insbesondere wird in einem Schritt S103 eine Eingabe eines Nutzers bzw. Patienten, ganz besonders die Betätigung der Startanzeige bzw. der Auslöseranzeige durch den Nutzer bzw. Patienten überwacht.

Die Überwachung der Eingabe führt vorzugsweise zu einer Entscheidung, ob die Startanzeige bzw. Auslöseranzeige betätigt worden ist, wie in Schritt S104 angedeutet ist.

Wird in Schritt S104 entschieden, dass eine Betätigung der Startanzeige bzw. Auslöseranzeige stattgefunden hat, werden vorzugsweise zwei parallele Zweige von der App bzw. dem Verfahren verfolgt. Zum einen wird in einem ersten Zweig Z1 vorzugsweise überwacht, ob eine visuelle Stoppanzeige (insbesondere auf dem Bildschirm 16) betätigt wird. Diese Überwachung führt vorzugsweise zu einer Entscheidung, ob die Stoppanzeige betätigt worden ist, wie in Schritt S105a angedeutet ist.

Zum anderen wird oder werden in einem zweiten Zweig Z2 parallel zur Überwachung der Stoppanzeige in einem Schritt S105 ein elektrischer Signalwert oder mehrere elektrische Signalwerte eines akustischen Signalaufnehmers, z. B. eines Mikrofons des portablen Kommunikationsgeräts 12, ausgelesen. Ein elektrischer Signalwert des akustischen Signalaufnehmers kann z. B. durch das erfindungsgemäße Verfahren erhalten werden. Vorzugsweise veranlasst die App bzw. das portable Kommunikationsgerät 12 im Schritt S105 eine Verarbeitung der elektrischen Signalwerte, insbesondere eine Digitalisierung und Speicherung der elektrischen Signalwerte.

In einem weiteren Schritt S106 im Zweig Z2 wird mit Hilfe der App bzw. des portablen Kommunikationsgeräts 12 ein beim Inhalationsvorgang erzeugter Volumenstrom bzw. Durchfluss bestimmt und/oder ein Durchflussprofil erstellt.

Innerhalb des Zweiges Z2 wird vorzugsweise das Starten eines Inhalationsvorgangs durch die App bzw. das portable Kommunikationsgerät 12 überwacht. Die Überwachung führt vorzugsweise zu einer Entscheidung, ob der Inhalationsvorgang gestartet wurde, wie in Schritt S107 angedeutet ist.

Dabei ist die App bzw. das portable Kommunikationsgerät 12 vorzugsweise so ausgelegt, dass ein Betätigen des Auslösers als Starten eines Inhalationsvorgangs interpretiert wird, was zu der Entscheidung führt, dass der Inhalationsvorgang gestartet wurde. Ein Betätigen des Auslösers kann beispielsweise ein akustisches Signal erzeugen. Dieses akustische Signal kann mittels des akustischen Signalaufnehmers aufgenommen und anschließend von der App bzw. dem portablen Kommunikationsgerät 12 ausgewertet werden.

Alternativ kann die App bzw. das portable Kommunikationsgerät 12 auch so ausgelegt sein, dass ein Betätigen der Auslöseranzeige als Starten eines Inhalationsvorgangs interpretiert wird.

Wird in Schritt S107 entschieden, dass ein Inhalationsvorgang gestartet wurde, wird zum einen in einem Schritt S108 vorzugsweise eine Startzeit von der App bzw. dem portablen Kommunikationsgerät 12 bestimmt. Daneben können vorzugsweise noch weitere Zeitwerte von der App bzw. dem portablen Kommunikationsgerät 12 über Zeitnehmer bestimmt werden, z. B. in einem Schritt S109.

Wird in Schritt S107 entschieden, dass ein Inhalationsvorgang gestartet wurde, wird zum anderen vorzugsweise das Beenden des Inhalationsvorgangs durch die App bzw. das portable Kommunikationsgerät 12 überwacht. Die Überwachung führt vorzugsweise zu einer Entscheidung, ob der Inhalationsvorgang beendet wurde, wie in Schritt S110 angedeutet ist.

Dabei ist die App bzw. das portable Kommunikationsgerät 12 vorzugsweise so ausgelegt, dass ein Loslassen des Auslösers als Beenden des Inhalationsvorgangs interpretiert wird, was zu der Entscheidung führt, dass der Inhalationsvorgang beendet wurde. Ein Loslassen des Auslösers kann beispielsweise ein akustisches Signal erzeugen. Dieses akustische Signal kann mittels des akustischen Signalaufnehmers aufgenommen und anschließend von der App bzw. dem portablen Kommunikationsgerät 12 ausgewertet werden.

Alternativ kann die App bzw. das portable Kommunikationsgerät 12 auch so ausgelegt sein, dass ein nochmaliges Betätigen der Auslöseanzeige oder ein Betätigen einer Beendigungsanzeige (insbesondere auf dem Bildschirm 16) als Beenden des Inhalationsvorgangs interpretiert wird.

Wird in Schritt S110 entschieden, dass der Inhalationsvorgang beendet wurde, wird in einem Schritt S111 vorzugsweise eine Stoppzeit von der App bzw. dem portablen Kommunikationsgerät 12 bestimmt.

Führt die Überwachung des Beendens des Inhalationsvorgangs nach Ablauf einer definierten Zeit (z. B. 20 Sekunden) ab festgestelltem Start des Inhalationsvorgangs nicht zu einer Entscheidung, dass der Inhalationsvorgang beendet wurde, erfolgt vorzugsweise eine Beendigung bzw. ein Abbruch der App bzw. des Ablaufs. Vorzugsweise wird dazu in einem Schritt S114 ein Abbruch überprüft. Wird von der App bzw. dem portablen Kommunikationsgerät 12 ein Abbruch festgestellt, wird die App bzw. der Ablauf in einem Schritt S115 beendet.

Im Schritt S115 kann beispielsweise die GUI beendet werden, so dass sie nicht mehr angezeigt wird. Ferner können im Schritt S115 Zeitwerte zurückgesetzt und/oder Speicher freigegeben werden.

Wurde in Schritt S111 eine Stoppzeit bestimmt, wird bei dem bevorzugten Verfahrensablauf in Fig. 8 von der App bzw. dem portable Kommunikationsgerät 12 in einem Schritt S112 vorzugsweise ein Zeitnehmer bestimmt. Außerdem wird vorzugsweise eine Auswertung der elektrischen Signalwerte vorgenommen. So kann in einem Schritt S116 beispielsweise mit Hilfe der App bzw. des portablen Kommunikationsgeräts 12 eine effektive Inhalationszeit und/oder inhalierte Medikamentendosis wie bereits beschrieben bestimmt werden.

Ergebnisse der Auswertung können auf der grafischen Benutzeroberfläche bzw. dem Bildschirm 16 angezeigt, wozu die grafische Benutzeroberfläche in einem Schritt S117 aktualisiert werden kann.

Ferner ist die App bzw. das portable Kommunikationsgerät 12 vorzugsweise so ausgeführt, dass eine Rückkopplung an den Patienten und/oder einen Dritten erfolgt, insbesondere eine Warnmeldung ausgegeben wird, wenn der durch die App bzw. das portable Kommunikationsgerät 12 bestimmte Durchfluss über einen Wert von ca. 40 Litern pro Minute steigt und/oder unter einen Wert von ca. 20 Litern pro Minute fällt.

Wurde in Schritt S105a entschieden, dass die Stoppanzeige betätigt worden ist, wird vorzugsweise die GUI im Schritt S117 aktualisiert und/oder im Schritt S114 ein Abbruch überprüft.

Die App bzw. der Trainingsvorgang kann auch durch Betätigung einer Abbruchanzeige (insbesondere auf dem Bildschirm 16) beendet bzw. abgebrochen werden.

Einzelne Schritte der App können auch unabhängig von anderen Schritten realisiert werden.

**Bezugszeichenliste:**

1  Inhalationstrainingssystem
2  Mundstück
3  Wandlereinrichtung
4  Lufteinlass
5  Einlasskanal
6  Drosselstelle
7  Resonanzraum
8  Kante
9  Auslasskanal
10  Tonauslass
11  Halteeinrichtung
12  elektronisches Gerät
13  Rückwand
14  oberer Haltebereich
15  unterer Haltebereich
16  Bildschirm

17  Bedienknopf / Mikrofon
18  Schutzkappe
19  Lagerung
20  Zuluftöffnung
21  Teleskopauszug

S  Schritt
Z1  erster Zweig
Z2  zweiter Zweig

**Patentansprüche**

1. Inhalationstrainingssystem (1) zum Trainieren eines Medikamenteninhalationsvorgangs eines Patienten,
mit einem Mundstück (2), in dem bei einem Inhalationsvorgang des Patienten eine Luftströmung auftritt, und
mit einer Wandlereinrichtung (3), die zur Umwandlung der Luftströmung in ein hörbares Pfeifen ausgebildet ist und dazu eine Pfeife umfasst,
**dadurch gekennzeichnet,**
**dass** das Inhalationstrainingssystem (1) ein portables Kommunikationsgerät (12) aufweist, das zur Umwandlung des Pfeifens in ein Mikrofonsignal mittels eines Mikrofons (17) des portablen Kommunikationsgeräts (12) ausgebildet ist und dass die Wandlereinrichtung (3) in und/oder an dem Mundstück angebracht ist.

2. Inhalationstrainingssystem nach Anspruch 1, **dadurch gekennzeichnet,**
**dass** die Wandlereinrichtung (3) auswechselbar ist.

3. Inhalationstrainingssystem nach Anspruch 1 oder 2, **dadurch gekennzeichnet, dass** das Mundstück (2) in oder auf die Wandlereinrichtung (3) gesteckt ist.

4. Inhalationstrainingssystem nach einem der vorhergehenden Ansprüche,
**dadurch gekennzeichnet, dass** das Inhalationstrainingssystem (1) eine Halteeinrichtung (11) aufweist, die zur lösbaren Halterung des portablen Kommunikationsgeräts (12) und zur Ausrichtung des portablen Kommunikationsgeräts (12) relativ zur Wandlereinrichtung (3) ausgebildet ist, so dass das portable Kommunikationsgerät (12) mittels des Mikrofons (17) das Pfeifen präzise aufnehmen kann.

5. Inhalationstrainingssystem nach Anspruch 4, **dadurch gekennzeichnet,**
**dass** das Mundstück (2), die Wandlereinrichtung (3) und die Halteeinrichtung (11) eine Baueinheit bilden.

6. Inhalationstrainingssystem nach einem der vorhergehenden Ansprüche,
**dadurch gekennzeichnet, dass** das portable Kommunikationsgerät (12) zur Bestimmung der effektiven Inhalationszeit ausgebildet ist.

7. Verfahren zum Trainieren eines Medikamenteninhalationsvorgangs eines Patienten ohne Abgabe einer Medikamentendosis, wobei eine bei einem Inhalationsvorgang des Patienten in einem Mundstück (2) auftretende Luftströmung durch eine in und/oder an dem Mundstück (2) angebrachten Wandlereinrichtung (3) umgewandelt wird, **wobei** die Luftströmung mittels einer Pfeife der Wandlereinrichtung (3) in ein hörbares Pfeifen umgewandelt wird, das Pfeifen mittels eines Mikrofons (17) eines portablen Kommunikationsgeräts (12) in ein Mikrofonsignal umgewandelt wird und das Mikrofonsignal in dem portablen Kommunikationsgerät (12) verarbeitet und/oder ausgewertet wird.

8. Verfahren nach Anspruch 7, **dadurch gekennzeichnet, dass** mit Hilfe des Mikrofonsignals von dem portablen Kommunikationsgerät (12) eine effektive Inhalationszeit, ein beim Inhalationsvorgang erzeugter Volumenstrom und/oder eine dabei erzeugte Strömungsgeschwindigkeit bestimmt oder geschätzt wird bzw. werden.

9. Verwendung eines portablen Kommunikationsgeräts zum Trainieren eines Medikamenteninhalationsvorgangs eines Patienten, wobei eine bei einem Inhalationsvorgang des Patienten in einem Mundstück (2) auftretende Luftströmung durch eine in und/oder an dem Mundstück (2) angebrachte Wandlereinrichtung (3) mit einer Pfeife in ein hörbares Pfeifen und dieses Pfeifen mittels eines Mikrofons (17) des portablen Kommunikationsgeräts (12) in ein Mikrofonsignal umgewandelt wird und wobei das Mikrofonsignal durch das portable Kommunikationsgerät (12) ausgewertet wird.

10. Verwendung eines portablen Kommunikationsgeräts gemäß Anspruch 9, **dadurch gekennzeichnet, dass** das Trainieren anhand eines Inhalationstrainingssystems (1) gemäß einem der Ansprüche 1 bis 6 und/oder anhand eines Verfahrens gemäß Anspruch 7 oder 8 durchgeführt wird.

11. Verwendung eines portablen Kommunikationsgeräts gemäß Anspruch 9 oder 10, **dadurch gekennzeichnet, dass** das Trainieren anhand einer Software durchgeführt wird, die über ein Online-Portal bezogen und installiert werden kann.

## Claims

1. Inhalation training system (1) for training of a drug inhalation process of a patient, with a mouthpiece (2) in which an air flow occurs in an inhalation process of the patient, and with a converter apparatus (3) which is made for conversion of the air flow into an audible whistling and comprises a whistle for this purpose, **characterized in that** the inhalation training system (1) comprises a portable communications device (12) which is designed to convert the whistling into a microphone signal by means of a microphone (17) of the portable communications device (12) and **in that** the converter apparatus (3) is located in and/or at the mouthpiece.

2. Inhalation training system according to claim 1, **characterized in that** the converter apparatus (3) is interchangeable.

3. Inhalation training system according to claim 1 or 2, **characterized in that** the mouthpiece (2) is plugged into or onto the converter apparatus (3).

4. Inhalation training system according to any one of the preceding claims, **characterized in that** the inhalation training system (1) has a holding apparatus (11) which is designed for detachable holding of the portable communications device (12) and for the alignment of the portable communications device (12) relative to the converter apparatus (3) so that the portable communications device (12) can precisely record the whistling by means of the microphone (17).

5. Inhalation training system according to claim 4, **characterized in that** the mouthpiece (2), the converter apparatus (3) and the holding apparatus (11) form a unit.

6. Inhalation training system according to any one of the preceding claims, **characterized in that** the portable communications device (12) is designed for determining the effective inhalation time.

7. Method for training of a drug inhalation process of a patient without delivery of a drug dose, an air flow which occurs in a mouthpiece (2) in an inhalation process of the patient being converted by a converter apparatus (3) located in and/or at the mouthpiece (2), the air flow being converted by means of a whistle of the converter apparatus (3) into an audible whistling, the whistling being converted by means of a microphone (17) of a portable communications device (12) into a microphone signal and the microphone signal being processed and/or evaluated in the portable communications device (12).

**8.** Method according to claim 7, **characterized in that** using the microphone signal an effective inhalation time, a volumetric flow produced during the inhalation process and/or a flow velocity generated in doing so are determined or estimated by the portable communications device (12).

**9.** Use of a portable communications device for training of a drug inhalation process of a patient, an air flow which occurs in a mouthpiece (2) in an inhalation process being converted into an audible whistling by a converter apparatus (3) with a whistle, the converter apparatus (3) being located in and/or at the mouthpiece (2), and said whistling being converted into a microphone signal by means of a microphone (17) of the portable communications device (12), and the microphone signal being evaluated by the portable communications device (12).

**10.** Use of a portable communications device according to claim 9, **characterized in that** the training is carried out using an inhalation training system (1) according to any one of claims 1 to 6 and/or using a method according to claim 7 or 8.

**11.** Use of a portable communications device according to claim 9 or 10, **characterized in that** the training is carried out using a software which can be ordered via an online portal and installed.

**Revendications**

**1.** Système d'entraînement à l'inhalation (1) pour l'entraînement à une procédure d'inhalation d'un médicament d'un patient, comprenant un embout (2) dans lequel un flux d'air pénètre lors d'une procédure d'inhalation du patient, et comprenant un dispositif de conversion (3) qui est conçu pour convertir le flux d'air en un sifflement audible et qui comporte à cet effet un sifflet, **caractérisé en ce que** le système d'entraînement à l'inhalation (1) comprend un appareil de communication portable (12) qui est conçu pour convertir le sifflement en un signal de microphone au moyen d'un microphone (17) de l'appareil de communication portable (12) et **en ce que** le dispositif de conversion (3) est monté dans et/ou sur l'embout.

**2.** Système d'entraînement à l'inhalation selon la revendication 1, **caractérisé en ce que** le dispositif de conversion (3) est interchangeable.

**3.** Système d'entraînement à l'inhalation selon la revendication 1 ou 2, **caractérisé en ce que** l'embout (2) est emboîté dans ou sur le dispositif de conversion (3).

**4.** Système d'entraînement à l'inhalation selon l'une quelconque des revendications précédentes, **caractérisé en ce que** le système d'entraînement à l'inhalation (1) comprend un dispositif de retenue (11) qui est conçu pour retenir de façon amovible l'appareil de communication portable (12) et pour orienter l'appareil de communication portable (12) par rapport au dispositif de conversion (3), de sorte que l'appareil de communication portable (12) puisse enregistrer le sifflement avec précision au moyen du microphone (17).

**5.** Système d'entraînement à l'inhalation selon la revendication 4, **caractérisé en ce que** l'embout (2), le dispositif de conversion (3) et le dispositif de retenue (11) forment une unité modulaire.

**6.** Système d'entraînement à l'inhalation selon l'une quelconque des revendications précédentes, **caractérisé en ce que** l'appareil de communication portable (12) est conçu pour déterminer le temps d'inhalation effectif.

**7.** Procédé d'entraînement à une procédure d'inhalation de médicament d'un patient sans délivrance d'une dose de médicament, dans lequel
un flux d'air pénétrant dans un embout (2) lors d'une procédure d'inhalation du patient est converti par un dispositif de conversion (3) monté dans et/ou sur l'embout (2),
le flux d'air est converti en un sifflement audible au moyen d'un sifflet du dispositif de conversion (3),
le sifflement est converti en signal de microphone au moyen d'un microphone (17) d'un appareil de communication portable (12) et
le signal de microphone est traité et/ou évalué dans l'appareil de communication portable (12).

**8.** Procédé selon la revendication 7, **caractérisé en ce qu'**un temps d'inhalation effectif, un débit volumique produit lors de la procédure d'inhalation et/ou une vitesse de flux produite à cette occasion sont déterminés ou estimés par l'appareil de communication portable (12) à l'aide du signal de microphone.

**9.** Utilisation d'un appareil de communication portable pour l'entraînement à une procédure d'inhalation de médicament d'un patient, un flux d'air pénétrant dans un embout (2) lors d'une procédure d'inhalation du patient étant converti en un sifflement audible par un dispositif de conversion (3) monté dans et/ou sur l'embout (2) au moyen d'un sifflet et ce sifflement étant converti en signal de microphone au moyen d'un microphone (17) de l'appareil de communication portable (12) et le signal de microphone étant évalué par l'appareil de communicatif portable (12).

**10.** Utilisation d'un appareil de communication portable selon la revendication 9, **caractérisé en ce que** l'entraînement est réalisé au moyen d'un système d'entraînement à l'inhalation (1) selon l'une quelconque des revendications 1 à 6 et/ou au moyen d'un procédé selon la revendication 7 ou 8.

**11.** Utilisation d'un appareil de communication portable selon la revendication 9 ou 10, **caractérisé en ce que** l'entraînement est réalisé au moyen d'un logiciel, qui peut être téléchargé et installé par l'intermédiaire d'un portail en ligne.

1

13

14

16

11

12

5

17

4

6

15

3

2

Fig. 1

1

14

11

13

III

15

III

3

Fig. 2

15

5

4

6

13

7

8   10   9

Fig. 3

Fig. 5

Fig. 4

Fig. 6

Fig. 7

Fig. 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2010023591 A2
- US 20120116241 A1
- GB 2479953 A
- EP 0933092 A1
- WO 9713553 A1
- US 5839429 A
- WO 0069496 A1
- US 2010192948 A1
- US 2009263773 A1
- WO 2011056889 A1
- US 20040187869 A1
- WO 2011153406 A2
- WO 2006008745 A2
- US 2011226236 A1
- WO 2007101438 A1